# EUROPEAN PATENT APPLICATION

(11) **EP 1 891 979 A1**
(43) Date of publication of application: **27.02.2008**
(21) Application number: 06270082.8
(22) Date of filing: 20.08.2006
(51) Int. Cl.: A61L 9/14

(54) **Mechanical freshener**

(71) Applicant: Hakhov, Vadym, 107 Carr road E17 5EP London (GB)
(72) Inventor: Hakhov, Vadym, 107 Carr road E17 5EP London (GB)

(57) **Abstract**

A mechanical freshener is simple device which starts work when it starts or stops moving.
A mechanical freshener fixes to the door. A mechanical freshener has the base 3 , a pendulum which comprise the bob 4 and the lever 8. The pendulum adapted to touch the button of can with freshener. When the door starts or stops moving the pendulum starts oscillating then the end 10 of the lever 8 starts pushing the button 11 of can 7 so the can starts spraying. The amount of pressure can be adjust.

## Description

The invention provided economical mechanical freshener.

Most of fresheners work continuously some other need impact to start working.
An object of this invention provides a mechanical freshener which starts to work when it starts or stops moving therefore if you fix mechanical freshener to the door you never forget refresh yours premises

Preferably a mechanical freshener can be made from plastic and metal materials.
Now invention will be described with reference to the accompanying drawing:

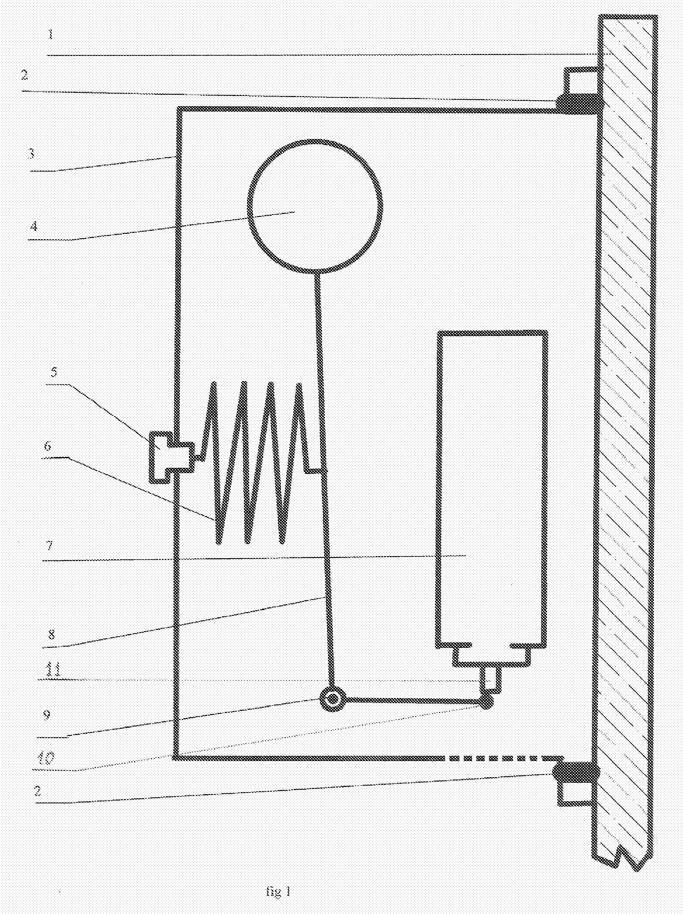

figure 1 shows a side view of mechanical freshener.

As shown in Figure 1, the case 3 fixes to the door 1 by fixers 2.
A pendulum including the bob 4 and the lever 8.
The bob 4 of pendulum fixes to the lever 8.
The lever 8 fixes to the case 3 by hinge 9.
The can of air freshener 7 fixes inside of the case 3 .
The end 10 touches the button11.

When the door 1 starts (or stops) moving the pendulum starts oscillations (displacement of the pendulum is not synchronised with the displacement of the door because the bob 4 has inertia) then the end 10 starts to push the button 11 of can 7 so the can starts spraying.

The spring 6 and the bolt 5 regulate the amount of pendulums oscillations.

## Claims

1. A mechanical freshener includes a base fixed to the door, a pendulum , a can of air freshener and mechanism to regulate the amount of pressure.
The pendulum adapted to push a button of can.

2. A mechanical freshener as claimed in claim 1 includes a pendulum which comprise a bob and a lever. The pendulum fixes inside the base by hinge

3. A mechanical freshener as claimed in claims 1 and 2 provided the pendulum which adapted starts oscillating when the door starts or stops moving.

4. A mechanical freshener as described herein with reference to Figure 1 of the accompanying drawing.
